# FASCICULE DE BREVET EUROPEEN

(11) **EP 4 251 317 B1**
(45) Date de publication et mention de la délivrance du brevet: **18.11.2026**
(21) Numéro de dépôt: 21824422.6
(22) Date de dépôt: 24.11.2021
(51) Int. Cl.: B01J 23/26, B01J 38/04, B01J 38/46, C07C 17/20, B01J 23/86

(54) **PROCÉDÉ DE TRAITEMENT D'UN CATALYSEUR AVANT DÉCHARGEMENT**
VERFAHREN ZUR BEHANDLUNG EINES KATALYSATORS VOR DEM ENTLADEN
METHOD FOR TREATING A CATALYST BEFORE UNLOADING

(30) Priorité: 27.11.2020 FR 2012271
(43) Date de publication de la demande: 04.10.2023
(73) Titulaire: ARKEMA FRANCE, 92800 Puteaux (FR)
(72) Inventeur: WENDLINGER, Laurent, 69491 PIERRE-BENITE CEDEX (FR); DEUR-BERT, Dominique, 69491 PIERRE-BENITE CEDEX (FR)
(74) Mandataire: Arkema Patent
(86) Numéro de dépôt international: PCT/FR2021/052079
(87) Numéro de publication internationale: WO 2022/112711

(56) Documents cités:
- EP-A1- 3 238 820
- WO-A1-2014/120493
- WO-A1-2018/178553

## Description

### Domaine technique de l'invention

La présente invention concerne un procédé de traitement d'un catalyseur. En particulier, la présente invention concerne un procédé de traitement d'un catalyseur avant son déchargement

### Arrière-plan technologique de l'invention

Les catalyseurs utilisés dans les réactions chimiques sont des matériaux essentiels pour la production de nombreux composés et ont pour fonction principale d'augmenter la vitesse des réactions. Les catalyseurs sont sélectionnés en fonction de la réaction à mettre en œuvre. Dans le domaine de la production de composés alcanes ou alcènes contenant des atomes d'halogènes, le catalyseur est généralement utilisé en phase gazeuse. Le catalyseur peut soit être utilisé en combinaison avec un halogénure d'hydrogène soit générer un halogénure d'hydrogène au cours de la mise en œuvre d'une réaction. A la fin de la mise en œuvre de la réaction, une partie de l'halogénure d'hydrogène est présent dans le réacteur et même absorbé ou adsorbé par le catalyseur lui-même. La présence de cet halogénure d'hydrogène est particulièrement dangereuse lorsque des opérations de maintenance nécessitant le déchargement du catalyseur doivent être mises en œuvre.

Il est donc nécessaire d'éliminer cet halogénure d'hydrogène du réacteur et du catalyseur pour garantir la sécurité des intervenants lors du déchargement du catalyseur. Le document EP 3 238 820 décrit un procédé de déchargement d'un catalyseur mettant en œuvre une étape de traitement à haute température. Cette étape de traitement à haute température tend à détériorer partiellement le catalyseur et consomme beaucoup d'énergie.

Il y a donc un besoin pour un procédé de déchargement du catalyseur sûr, efficace et peu couteux en énergie.

### Résumé de l'invention

La présente invention concerne un procédé de traitement, dans un réacteur contenant un lit catalytique, d'un catalyseur solide, ledit procédé comprenant les étapes :
a) Mise en œuvre dans ledit réacteur d'une réaction catalytique en phase gazeuse à une température du lit catalytique T1 en présence d'un halogénure d'hydrogène ou générant la formation d'un halogénure d'hydrogène,
b) Ecoulement d'un gaz inerte à travers le lit catalytique à une température du lit catalytique T2 inférieure à T1 ; la température T2 étant supérieure à 30°C.

Le présent procédé apporte un gain économique et énergétique puisque le gaz inerte s'écoule à travers le lit catalytique à une température plus faible que la température de la réaction catalytique. En outre, en mettant en œuvre l'étape b) à une température T2 inférieure à la température T1 à laquelle la réaction catalytique de l'étape a) est mise en œuvre, le catalyseur ne se détériore pas, ce qui permet une utilisation ultérieure de celui-ci, éventuellement après une régénération, sans perte d'activité. Dans le présent procédé, la mise en œuvre de l'étape b) est postérieure, de préférence subséquente, à la mise en œuvre de l'étape a).

Selon un mode de réalisation préféré, le gaz inerte introduit dans le réacteur est à une température comprise entre la température ambiante et la température T2. De préférence, le gaz inerte introduit dans le réacteur est à température ambiante. Le gaz inerte introduit va ainsi progressivement refroidir le lit catalytique tout en éliminant les résidus d'halogénure d'hydrogène présents dans le réacteur et adsorbés ou absorbés dans le catalyseur. L'introduction d'un gaz inerte dont la température est la température ambiante ou proche de la température ambiante permet un gain énergétique et économique supplémentaire.

Selon un mode de réalisation préféré, l'halogénure d'hydrogène est le fluorure d'hydrogène ou le chlorure d'hydrogène.

Selon un mode de réalisation préféré, la température T2 diminue au cours de la mise en œuvre de l'étape b), de préférence la température T2 diminue à une vitesse inférieure à 1°C/min au cours de la mise en œuvre de l'étape b).

Selon un mode de réalisation préféré, le gaz inerte s'écoule à un débit supérieur à 0,1 ml/min par ml de catalyseur.

Selon un mode de réalisation préféré, le catalyseur est à base de carbone ou à base d'un métal sélectionné parmi le groupe consistant en Cr, Fe, Sb, Ni, Co, Zn, Al et Mn.

Selon un mode de réalisation préféré, l'étape a) met en œuvre une réaction en phase gazeuse entre HF et un composé hydrohalocarboné en C₁-C₄ **A** ou l'étape a) met en œuvre une réaction de déshydrohalogénation en phase gazeuse d'un composé hydrocarboné saturé en C₁-C₄ **B** comprenant au moins un atome d'halogène pour former un composé hydrocarboné insaturé en C₁-C₄ et un halogénure d'hydrogène.

Selon un mode de réalisation préféré, le composé **A** est sélectionné parmi le groupe consistant en 1,1,2-trichloroéthane, 2-chloro-1,1,1-trifluoroéthane , 1-chloro-1,1,2-trifluoroéthane, 1-chloro-1,2,2-trifluoroéthane, 1,1,1,3,3,3-hexachlorodifluoropropane, 1,1,1,3,3,3-hexachloropropane, 1,1,1,3,3-pentachloropropane, 2,2,3-trichloro-1,1,1,3,3-pentafluoropropane, 1,1,1,3,3,3-hexachlorodifluoropropane, 1,1-dichloro-2,2,3,3,3-pentafluoropropane, 1,3-dichloro-1,2,2,3,3-pentafluoropropane, 1,1,1,2,3-pentachloropropane, 1,1,2,2,3-pentachloropropane, 1,1,1,3,3-pentachloropropane, 1,2-dichloroéthylène, 1,1,2-trichloroethylène, 1,1,2,2-tétrachloroéthylène, 1,1,2-trichloro-3,3,3-trifluoropropène, l'hexafluoropropène, 1,1,3,3,3-pentafluoropropène, 1,3,3,3-tétrafluoropropène, 2-chloro-3,3,3-trifluoropropène, 1,1,2,3-tétrachloropropène, 2,3,3,3-tétrachloropropène, 1-chloro-3,3,3-trifluoropropène, 1,1,3,3-tétrachloropropène, 1,3,3,3-tétrachloropropène, 2,3-dichloro-1,1,1-trifluoropropène, 2-chloro-1,1,1,2-tétrafluoropropane ; ou
le composé **B** est sélectionné parmi le groupe consistant en 1,1-difluoroéthane, 1,1,1-trifluoroéthane, 1,1,1,2,2-pentafluoroéthane, 2-chloro-1,1,1-trifluoroéthane, 1,1,1,2-tétrafluoroéthane, 1,1,2,2-tétrafluoroéthane, 1,1,1,2-tétrafluoropropane, 1,1,1,2,3,3-hexafluoropropane, 1,1,1,3,3,3-hexafluoropropane, 1,1,1,2,3,3-hexafluoropropane, 1,1,1,2,2-pentafluoropropane, 1,1,1,2,3-pentafluoropropane, 1,1,1,3,3-pentafluoropropane, 2,3-dichloro-1,1,1-trifluoropropane, 2-chloro-1,1,1,2-tétrafluoropropane.

Selon un mode de réalisation préféré, ledit procédé comprend une étape c) de déchargement du catalyseur dudit réacteur.

Selon un mode de réalisation préféré, l'étape b) comprend une étape b1) de refroidissement du lit catalytique de la température T1 à T2 puis une étape b2) d'écoulement dudit gaz inerte à travers le lit catalytique.

La présente invention concerne également un procédé de traitement, dans un réacteur contenant un lit catalytique, d'un catalyseur solide, ledit procédé comprenant les étapes :
a) Mise en œuvre dans ledit réacteur d'une réaction catalytique en phase gazeuse à une température du lit catalytique T1 en présence d'un halogénure d'hydrogène ou générant la formation d'un halogénure d'hydrogène,
b) Ecoulement d'un gaz inerte à travers le lit catalytique à une température du lit catalytique T2 inférieure à T1 ; la température T2 diminuant au cours de la mise en œuvre de l'étape b).

### Description détaillée de la présente invention

La présente invention concerne un procédé de traitement d'un catalyseur. En particulier, la présente invention concerne un procédé de traitement d'un catalyseur solide. Ainsi, la présente invention concerne un procédé de traitement, dans un réacteur contenant un lit catalytique, d'un catalyseur solide.

De préférence, le présent procédé comprend les étapes :
a) Mise en œuvre dans ledit réacteur d'une réaction catalytique en phase gazeuse à une température du lit catalytique T1 en présence d'un halogénure d'hydrogène ou générant la formation d'un halogénure d'hydrogène,
b) Ecoulement d'un gaz inerte à travers le lit catalytique à une température du lit catalytique T2 inférieure à T1 ; la température T2 étant supérieure à 30°C.

Le procédé selon la présente invention est mené typiquement dans un réacteur muni d'un lit catalytique fixe. Le réacteur et ses lignes d'alimentation associées, lignes d'effluent et appareils associés doivent être construits dans des matériaux résistants aux halogénures d'hydrogène tels que le fluorure d'hydrogène ou le chlorure d'hydrogène. Les matériaux de construction typiques, bien connus en l'état de l'art de la fluoration, incluent les aciers inox, en particulier de type austénitique, les alliages bien connus à haute teneur en nickel, comme les alliages nickel-cuivre Monel^{®}, les alliages à base de nickel Hastelloy^{®} et les alliages nickel-chrome Inconel^{®}. Selon un mode de réalisation préféré, à l'étape a), l'halogénure d'hydrogène est sous forme anhydre. De préférence, le composé **A** et le composé **B** décrits ci-dessous peuvent être également sous forme anhydre.

Selon un mode de réalisation préféré, à l'étape b), le gaz inerte est sous forme anhydre.

Le terme anhydre se réfère à une teneur massique en eau inférieure à 1000 ppm, avantageusement 500 ppm, de préférence inférieure à 200 ppm, en particulier inférieure à 100 ppm, plus particulièrement inférieure à 50 ppm, de manière privilégiée inférieure à 25 ppm dans le composé considéré.

### Catalyseur

Selon un mode de réalisation préféré, le catalyseur est à base de carbone ou d'un métal sélectionné parmi le groupe consistant en Cr, Ti, Al, Mn, Ni, Co, Fe, Cu, Zn, Sn, Au, Ag, Pt, Pd, Ru, Rh, Mo, Zr, Ge, Nb, Ta, Ir, Hf, V, Mg, Li, Na, K, Ca, Cs, Ru et Sb ; de préférence le catalyseur est à base d'un métal sélectionné parmi le groupe consistant en Cr, Fe, Sb, Ni, Co, Zn, Al et Mn. Le catalyseur à base de carbone peut être du carbone activé, du charbon ou du graphite. Le catalyseur à base d'un métal peut être sous la forme d'oxyde, d'halogénure ou d'oxyhalogénure dudit métal. En particulier, le catalyseur est à base de Cr, AI, Fe ou Sb. Le catalyseur peut être un halogénure d'antimoine, de fer ou d'aluminium tel que SbCl₅, FeCl₃, ou AlCl₃. Le catalyseur peut être un oxyde de chrome, un oxyfluorure de chrome ou un fluorure de chrome. Lorsque le catalyseur est à base de chrome, celui-ci peut contenir un co-catalyseur sélectionné parmi le groupe consistant en Co, Zn, Mn, Ni ou un mélange de ceux-ci, dans une teneur massique de 1 à 10% sur base du poids total dudit catalyseur.

Ledit catalyseur peut être massique ou supporté. Le support peut être sélectionné parmi le groupe consistant en carbone activé, alumine et fluorure d'aluminium. Par exemple, des catalyseurs comme Cr₂O₃, MgF₂, SbCl₅ ou FeCl₃ peuvent être supportés sur du carbone activé. Ledit catalyseur peut être activé avant la mise en œuvre de l'étape a) détaillée ci-dessous. L'activation du catalyseur peut être mise en œuvre suivant les méthodes connues de l'homme du métier. Par exemple, l'activation du catalyseur peut être mise en œuvre en présence d'oxygène, d'HF ou d'azote ou un mélange de ceux-ci à une température comprise entre 100°C et 500°C.

### Etape a)

Ladite étape a) comprend la mise en œuvre dans ledit réacteur d'une réaction catalytique en phase gazeuse à une température du lit catalytique T1. Ladite réaction catalytique peut être soit mise en œuvre en présence d'un halogénure d'hydrogène soit générer la formation d'un halogénure d'hydrogène.

L'halogénure d'hydrogène peut être sélectionné parmi le groupe consistant en HF, HCl, HBr et HI. De préférence, l'halogénure d'hydrogène est le fluorure d'hydrogène (HF) ou le chlorure d'hydrogène (HCl).

Selon un mode de réalisation préféré, l'étape a) peut mettre en œuvre une réaction en phase gazeuse entre HF et un composé hydrohalocarboné **A** en C₁-C₄. De préférence, l'étape a) met en œuvre une réaction entre le fluorure d'hydrogène et un composé **A** pour former un composé hydrohalocarboné comprenant au moins un atome de fluor. Ledit composé **A** peut être un composé saturé de formule CH₂Cl₂, CH₂Br₂, CHCl₃, CCl_{4,} C₂Cl₆, C₂BrCl₅, C₂Cl₅F, C₂Cl₄F₂, C₂Cl₃F₃, C₂Cl₂F₄, C₂ClF₅, C₂HCl₅, C₂HCl₄F, C₂HCl₃F₂, C₂HCl₂F₃, C₂HClF₄, C₂HBrF₄, C₂H₂Cl₄, C₂H₂Cl₃F, C₂H₂Cl₂F₂, C₂H₂ClF₃, C₂H₃Cl₃**,** C₂H₃Cl₂F**,** C₂H₃ClF₂, C₂H₄Cl₂, C₂H₄ClF, C₃Cl₆F₂, C₃Cl₅F₃, C₃Cl₄F₄, C₃Cl₃F₅, C₃HCl₇, C₃HCl₆F, C₃HCl₅F₂, C₃HCl₄F₃, C₃HCl₃F₄, C₃HCl₂F₃, C₃Cl₂F₆, C₃H₂Cl₆, C₃H₂BrCl₅, C₃H₂Cl₅F, C₃H₂Cl₄F₂, C₃H₂Cl₃F₃, C₃H₂Cl₂F₄, C₃H₂ClF₃, C₃H₃Cl₅, C₃H₃Cl₄F, C₃H₃Cl₃F₂, C₃H₃Cl₂F₃, C₃H₃ClF₄, C₃H₄Cl₄, C₄Cl₄Cl₄, C₄Cl₄Cl₆**,** C₄H₆Cl₆**,** C₄H₅Cl₄F₁ et C₆H₄Cl₈ ou un composé insaturé de formule C₂Cl₄, C₂BrCl₃, C₂Cl₃F, C₂Cl₂F₂, C₂ClF₃, C₂F₄, C₂HCl₃, C₂HBrCl₂, C₂HCl₂F, C₂HClF₂, C₂HF₃, C₂H₂Cl₂, C₂H₂ClF, C₂H₂F₂, C₂H₃Cl, C₂H₃F, C₂H₄, C₃H₆, C₃H₅Cl**,** C₃H₄Cl₂**,** C₃H₃Cl₃**,** C₃H₂Cl₄**,** C₃HCl₅**,** C₃H₂ClF₃, C₃F₃HCl₂, C₃F₂H₂Cl₂, C₃F₄H, ClC₃Cl₆, C₃Cl₅F, C₃Cl₄F₂, C₃Cl₃F₃, C₃Cl₂F₄, C₃ClF₅, C₃HF₅, C₃H₂F₄, C₃F₆, C₄Cl₈, C₄Cl₂F₆, C₄ClF₇, C₄H₂F₆, et C₄HClF₆.

De préférence, ledit composé **A** peut être sélectionné parmi le groupe consistant en 1,1,2-trichloroéthane, 2-chloro-1,1,1-trifluoroéthane , 1-chloro-1,1,2-trifluoroéthane, 1-chloro-1,2,2-trifluoroéthane, 1,1,1,3,3,3-hexachlorodifluoropropane, 1,1,1,3,3,3-hexachloropropane, 1,1,1,3,3-pentachloropropane, 2,2,3-trichloro-1,1,1,3,3-pentafluoropropane, 1,1,1,3,3,3-hexachlorodifluoropropane, 1,1-dichloro-2,2,3,3,3-pentafluoropropane, 1,3-dichloro-1,2,2,3,3-pentafluoropropane, 1,1,1,2,3-pentachloropropane, 1,1,2,2,3-pentachloropropane, 1,1,1,3,3-pentachloropropane, 1,2-dichloroéthylène, 1,1,2-trichloroethylène, 1,1,2,2-tétrachloroéthylène, 1,1,2-trichloro-3,3,3-trifluoropropène, l'hexafluoropropène, 1,1,3,3,3-pentafluoropropène, 1,3,3,3-tétrafluoropropène, 2-chloro-3,3,3-trifluoropropène, 1,1,2,3-tétrachloropropène, 2,3,3,3-tétrachloropropène, 1-chloro-3,3,3-trifluoropropène, 1,1,3,3-tétrachloropropène, 1,3,3,3-tétrachloropropène, 2,3-dichloro-1, 1, 1-trifluoropropène, 2-chloro-1,1,1,2-tétrafluoropropane.

Plus préférentiellement, ledit composé **A** peut être sélectionné parmi le groupe consistant en 1,1,2-trichloroéthane, 2-chloro-1,1,1-trifluoroéthane , 1-chloro-1,1,2-trifluoroéthane, 1-chloro-1,2,2-trifluoroéthane, 2-chloro-3,3,3-trifluoropropène, 1,1,1,2,3-pentachloropropane, 1,1,2,2,3-pentachloropropane, 1,1,2,3-tétrachloropropène, 2,3,3,3-tétrachloropropène, 1-chloro-3,3,3-trifluoropropène, 1,1,1,3,3-pentachloropropane, 1,1,3,3-tétrachloropropène, 1,3,3,3-tétrachloropropène, 2,3-dichloro-1,1,1-trifluoropropène, 2-chloro-1,1,1,2-tétrafluoropropane ou leurs mélanges.

En particulier, des exemples spécifiques de réaction entre HF et le composé **A** incluent la conversion du 1,1,2-trichloroéthane (CHCl₂CH₂Cl ou HCC-140) en 1-chloro-2,2-difluoroéthane (CH₂ClCF₂H ou HCFC-142), la conversion du 1,1,1,3,3,3-hexachlorodifluoropropane (CCl₃CF₂CCl₃ ou CFC-212ca) en un mélange de 1,1,3-trichloro-1,2,2,3,3-pentafluoropropane (CCl₂FCF₂CClF₂ ou CFC-215ca) et de 1,3-dichloro-1,1,2,2,3,3-hexafluoropropane (CClF₂CF₂CClF₂ ou CFC-216ca), la conversion du 1,1,1,3,3,3-hexachloropropane (CCl₃CH₂CCl₃ ou HCC-230fa) en 1-chloro-1,1,3,3,3-pentafluoropropane (CF₃CH₂CClF₂ ou HCFC-235fa) et 1,1,1,3,3,3-hexafluoropropane (CF₃CH₂CF₃ ou HFC-236fa), la conversion du 1,1,1,3,3-pentachloropropane (CCl₃CH₂CHCl₂ ou HCC-240fa) en un mélange de 1,1,1,3,3-pentafluoropropane (CHF₂CH₂CF₃ ou HFC-245fa), 1-chloro-3,3,3-trifluoro-1-propène (CHCl=CHCF₃ ou HCFO-1233zd) et 1,3,3,3-tétrafluoropropène (CHF=CHCF₃ ou HFO-1234ze), la conversion du 2,2,3-trichloro-1,1,1,3,3-pentafluoropropane (CF₃CCl₂CClF₂ ou CFC-215aa) en un mélange de 1,1,1,3,3,3-hexachlorodifluoropropane (CF₃CCl₂CF₃ ou CFC-212ca) et 2-chloro-1,1,1,2,3,3,3-heptafluoropropane (CF₃CClFCF₃ ou CFC-217ba), la conversion du 1,1,1,3,3,3-hexachlorodifluoropropane (CF₃CCl₂CF₃ ou CFC-212ca) en 2-chloro-1,1,1,2,3,3,3-heptafluoropropane (CF₃ClFCF₃ ou CFC-217ba), la conversion d'un mélange contenant du 1,1-dichloro-2,2,3,3,3-pentafluoropropane (CF₃CF₂CHCl₂ ou HCFC-225ca) et 1,3-dichloro-1,2,2,3,3-pentafluoropropane (CClF₂CF₂CHClF ou HCFC-225cb) en un mélange de 1-chloro-1,2,2,3,3,3-hexafluoropropane (CF₃CF₂CHClF ou HCFC-226ca) et 1,1,1,2,2,3,3-heptafluoropropane (CF₃CF₂CHF₂ ou HFC-227ca), la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf), la conversion du 1,1,2,2,3-pentachloropropane (CHCl₂CCl₂CH₂Cl ou HCC-240aa) en 2-chloro-3,3,3-trifluoro-1-propène (CF₃CCl=CH₂ ou HCFO-1233xf), la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf), la conversion du 1,1,2,2,3-pentachloropropane (CHCl₂CCl₂CH₂Cl ou HCC-240aa) en 2,3,3,3-tétrafluoropropène (CF₃CF=CH₂ ou HFO-1234yf), la conversion du 1,1,1,3,3-pentachloropropane (CCl₃CH₂CHCl₂ ou HCC-240fa) en 1,3,3,3-tétrafluoropropène (CF₃CH=CHF ou HFO-1234ze), la conversion du 1,2-dichloroéthylène (CHCl=CClH ou HCO-1130) en 1-chloro-2,2-difluoroéthane (CH₂ClCF₂H ou HCFC-142)₂, la conversion du 1,1,2-trichloro-3,3,3-trifluoro-1-propène (CCl₂=CClCF₃ ou CFC-1213xa) en un mélange de 2,3-dichloro-1,1,1,3,3-pentafluoropropane (CF₃CHClCClF₂ ou HCFC-225da), de 2-chloro-1,1,1,3,3,3-hexafluoropropane (CF₃CHClCF₃ ou HCFC-226da) et/ou de 2-chloro-1,1,3,3,3-pentafluoro-1-propène (CF₃CCl=CF₂ ou CFC-1215xc), la conversion de l'hexafluoropropène (CF₃CF=CF₂ ou CFC-1216yc) en 1,1,1,2,3,3,3-heptafluoropropane (CF₃CHFCF₃ ou HFC-227ea), la conversion du 1,1,3,3,3-pentafluoropropène (CF₃CH=CF₂ ou HFO-1225zc) en 1,1,1,3,3,3-hexafluoropropane (CF₃CH₂CF₃ ou HFC-236fa), la conversion du 1,3,3,3-tétrafluoropropène (CF₃CH=CHF ou HFO-1234ze) en 1,1,1,3,3-pentafluoropropane (CF₃CH₂CHF₂ ou HFC-245fa), la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf), la conversion du 1,1,2,3-tétrachloro-1-propène (CCl₂=CClCH₂Cl ou HCO-1230xa) en 2-chloro-3,3,3-trifluoro-1-propène (CF₃CCl=CH₂ ou HCFO-1233xf) ou 2,3,3,3-tétrafluoropropène (CF₃CF=CH₂ ou HFO-1234yf), la conversion du 2,3,3,3-tétrachloro-1-propène (CCl₃CCl=CH₂ ou HCO-1230xf) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 1-chloro-3,3,3-trifluoro-1-propène (CF₃CH=CHCl ou HCFO-1233zd) ou du 1,1,3,3-tétrachloro-1-propène (CCl₂=CHCHCl₂ ou HCO-1230za) ou du 1,3,3,3-tétrachloroprop-1-ène (CCl₃CH=CHCl ou HCO-1230zd) en 1,3,3,3-tétrafluoropropène (CF₃CH=CHF ou HFO-1234ze), la conversion du 2,3-dichloro-1,1,1-trifluoropropène (CF₃CHCICH₂Cl ou HCFC-243db) en 2,3,3,3-tétrafluoropropène (CF₃CF=CH₂ ou HFO-1234yf), la conversion du 2-chloro-1,1,1,2-tétrafluoropropane (CF₃CFClCH₃ ou HCFC-244bb) en 1,1,1,2,2-pentafluoropropane (CF₃CF₂CH₃ ou HFC-245cb), la conversion du 1,1,2,2-tétrachloroéthylène (Cl₂C=CCl₂ ou PER) en 1,1,1,2,2-pentafluoroéthane (CF₃CF₂H ou HFC-125), la conversion du 2-chloro-1,1,1-trifluoroéthane (CF₃CH₂Cl ou R-133a) en 1,1,1,2-tétrafluoroéthane (CF₃CCH₂F ou R-134a), la conversion du 1,1,2,2-tétrachloroéthylène (Cl₂C=CCl₂ ou PER) en 1,1,1,2-tétrafluoroéthane (CF₃CCH₂F ou R-134a), la conversion du 1,1,2-trichloroéthylène (ClHC=CCl₂) en 1,1,1,2-tétrafluoroéthane (CF₃CCH₂F ou R-134a) et/ou 1,1,1,2,2-pentafluoroéthane (CF₃CF₂H ou HFC-125).

Plus particulièrement, des exemples spécifiques de réactions de fluoration de composés **A** incluent la conversion du 1,1,1,2,3-pentachloropropane (CCl₃CHClCH₂Cl ou HCC-240db) en 2-chloro-3,3,3-trifluoro-1-propène (CF₃CCl=CH₂ ou HCFO-1233xf), la conversion du 1,1,2,2,3-pentachloropropane (CHCl₂CCl₂CH₂Cl ou HCC-240aa) en 2-chloro-3,3,3-trifluoro-1-propène (CF₃CCl=CH₂ ou HCFO-1233xf), la conversion du 1,1,1,2,3-pentachloropropane (CCl₃CHClCH₂Cl ou HCC-240db) en 2,3,3,3-tétrafluoropropène (CF₃CF=CH₂ ou HFO-1234yf), la conversion du 1,1,2,2,3-pentachloropropane (CHCl₂CCl₂CH₂Cl ou HCC-240aa) en 2,3,3,3-tétrafluoropropène (CF₃CF=CH₂ ou HFO-1234yf), la conversion du 1,1,1,3,3-pentachloropropane (CCl₃CH₂CHCl₂ ou HCC-240fa) en 1,3,3,3-tétrafluoropropène (CF₃CH=CHF ou HFO-1234ze), la conversion du 1,1,2-trichloroéthane (CHCl₂CH₂Cl ou HCC-140) en 1-chloro-2,2-difluoroéthane (CH₂ClCF₂H ou HCFC-142), la conversion du 2-chloro-3,3,3-trifluoro-1-propène (CF₃CCl=CH₂ ou HCFO-1233xf) en 2,3,3,3-tétrafluoropropène (CF₃CF=CH₂ ou HFO-1234yf), la conversion du 1,1,2,3-tétrachloro-1-propène (CCl₂=CClCH₂Cl ou HCO-1230xa) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 2,3,3,3-tétrachloro-1-propène (CCl₃Cl=CH₂ ou HCO-1230xf) en 2-chloro-3,3,3-trifluoro-1-propène (CF₃CCl=CH₂ ou HCFO-1233xf) ou en 2,3,3,3-tétrafluoropropène (CF₃CF=CH₂ ou HFO-1234yf), la conversion du 1-chloro-3,3,3-trifluoro-1-propène (CF₃CH=CHCl ou HCFO-1233zd) ou du 1,1,3,3-tétrachloro-1-propène (CCl₂=CHCHCl₂ ou HCO-1230za) ou du 1,3,3,3-tétrachloroprop-1-ène (CCl₃CH=CHCl ou HCO-1230zd) en 1,3,3,3-tétrafluoropropène (CF₃CH=CHF ou HFO-1234ze, la conversion du 1,2-dichloroéthylène (CHCl=CClH ou HCO-1130) en 1-chloro-2,2-difluoroéthane (CH₂ClCF₂H ou HCFC-142), la conversion du 2,3-dichloro-1,1,1-trifluoropropène (CF₃CHCICH₂Cl ou HCFC-243db) en 2,3,3,3-tétrafluoropropène (CF₃CF=CH₂ ou HFO-1234yf), la conversion du 2-chloro-1,1,1,2-tétrafluoropropane (CF₃CFClCH₃ ou HCFC-244bb) en 1,1,1,2,2-pentafluoropropane (CF₃CF₂CH₃ ou HFC-245cb), la conversion du 1,1,2,2-tétrachloroéthylène (Cl₂C=CCl₂ ou PER) en 1,1,1,2,2-pentafluoroéthane (CF₃CF₂H ou HFC-125), la conversion du 2-chloro-1,1,1-trifluoroéthane (CF₃CH₂Cl ou R-133a) en 1,1,1,2-tétrafluoroéthane (CF₃CCH₂F ou R-134a), la conversion du 1,1,2,2-tétrachloroéthylène (Cl₂C=CCl₂ ou PER) en 1,1,1,2-tétrafluoroéthane (CF₃CCH₂F ou R-134a), la conversion du 1,1,2-trichloroéthylène (ClHC=CCl₂) en 1,1,1,2-tétrafluoroéthane (CF₃CCH₂F ou R-134a) et/ou 1,1,1,2,2-pentafluoroéthane (CF₃CF₂H ou HFC-125).

Selon un autre mode de réalisation préféré, l'étape a) peut mettre en œuvre une réaction de déshydrohalogénation en phase gazeuse d'un composé hydrocarboné saturé en C₁-C₄ **B** comprenant au moins un atome d'halogène pour former un composé hydrocarboné insaturé en C₁-C₄ et un halogénure d'hydrogène. De préférence, le composé **B** est sélectionné parmi le groupe consistant en 1,1-difluoroéthane, 1,1,1-trifluoroéthane, 2-chloro-1,1,1-trifluoroéthane, 1,1,1,2-tétrafluoroéthane, 1,1,2,2-tétrafluoroéthane, 1,1,1,2,2-pentafluoroéthane, 1,1,1,2-tétrafluoropropane, 1,1,1,3,3-pentafluoropropane, 1,1,1,2,3,3-hexafluoropropane, 1,1,1,3,3,3-hexafluoropropane, 1,1,1,2,3,3-hexafluoropropane, 1,1,1,2,2-pentafluoropropane, 1,1,1,2,3-pentafluoropropane, 2,3-dichloro-1,1,1-trifluoropropène et 2-chloro-1,1,1,2-tétrafluoropropane. En particulier, le composé **B** est sélectionné parmi le groupe consistant en 1,1,1,2,2-pentafluoropropane, 1,1,1,3,3-pentafluoropropane, 2,3-dichloro-1,1,1-trifluoropropène et 2-chloro-1,1,1,2-tétrafluoropropane. De préférence, l'halogénure d'hydrogène est HF ou HCl.

Des exemples spécifiques de déshydrohalogénation en phase gazeuse du composé **B** incluent la conversion du 1,1-difluoroéthane (CHF₂CH₃ ou HFC-152a) en chlorure de vinyle (CHF=CH₂ ou HFO-1141), la conversion du 1,1,1-trifluoroéthane (CF₃CH₃ ou HFC-143a) en fluorure de vinylidène (CF₂=CH₂ ou HFO-1132a), la conversion du 2-chloro-1,1,1-trifluoroéthane (CF₃CH₂Cl ou HCFC-133a) en 2-chloro-1,1-difluoroéthylène (CF₂=CHCl ou HCFO-1122), la conversion du 1,1,1,2-tétrafluoroéthane (CF₃CH₂F ou HFC-134a) en trifluoroéthylène (CF₂=CHF ou HFO-1123), la conversion du 1,1,2,2-tétrafluoroéthane (CHF₂CHF₂ ou HFC-134) en trifluoroéthylène (CF₂=CHF ou HFO-1123), la conversion du 1,1,1,2-tétrafluoropropane (CH₃CHFCF₃ ou HFC-254eb) en 1,1,1-trifluoropropène (CH₂=CHCF₃ ou HFO-1243zf), la conversion du 1,1,1,3,3-pentafluoropropane (CHF₂CH₂CF₃ ou HFC-245fa) en 1,3,3,3-tétrafluoropropène (CHF=CHCF₃ ou HFO-1234ze), la conversion du 1,1,1,2,3,3-hexafluoropropane (CHF₂CHFCF₃ ou HFC-236ea) en 1,2,3,3,3-pentafluoropropène (CHF=CFCF₃ ou HFO-1225ye), la conversion du 1,1,1,3,3,3-hexafluoropropane (CF₃CH₂CF₃ ou HFC-236fa) en 1,1,3,3,3-pentafluoropropène (CF₃CH=CF₂ ou HFO-1225zc), la conversion du 1,1,1,2,3,3-hexafluoropropane (CF₃CF₂CFH₂ ou HFC-236cb) en 1,2,3,3,3-pentafluoropropène (CHF=CFCF₃ ou HFO-1225ye), la conversion du 1,1,1,2,2-pentafluoropropane (CF₃CF₂CH₃ ou HFC-245cb) en 2,3,3,3-tétrafluoropropène (CF₃CF=CH₂ ou HFO-1234yf) et la conversion du 1,1,1,2,3-pentafluoropropane (CF₃CHFCH₂F ou HFC-245eb) en 2,3,3,3-tétrafluoropropène (CF₃CF=CH₂ ou HFO-1234yf), la conversion du (CF₃CHCICH₂Cl ou HCFC-243db) en 2-chloro-3,3,3-trifluoro-1-propène (CF₃CCl=CH₂ ou HCFO-1233xf), la conversion du 2-chloro-1,1,1,2-tétrafluoropropane (CF₃CFClCH₃ ou HCFC-244bb) en 2,3,3,3-tétrafluoropropène (CF₃CF=CH₂ ou HFO-1234yf).

La température du lit catalytique T1 peut être comprise entre 100°C et 500°C, avantageusement entre 150°C et 450°C, de préférence entre 200°C et 400°C, plus préférentiellement entre 250°C et 380°C.

L'étape a) peut également être mise en œuvre suivant les conditions opératoires suivantes :
- un rapport molaire HF/composé hydrocarbure entre 1:1 et 150:1, de préférence entre 2:1 et 125:1, plus préférentiellement entre 3:1 et 100:1 ;
- un temps de contact entre 1 et 100 s, de préférence entre 2 et 75 s, en particulier entre 3 et 50 s ;
- une pression entre la pression atmosphérique et 20 bar, de préférence entre 2 et 18 bar, plus préférentiellement entre 3 et 15 bar.

L'homme du métier adaptera les conditions opératoires ci-dessus en fonction de la réaction mise en œuvre à l'étape a).

L'étape a) peut être mise en œuvre sur une durée comprise entre 2000 et 25000 h, de préférence entre 2500 et 24000 h, plus préférentiellement entre 3000 et 20000 h.

On peut ajouter un oxydant, comme l'oxygène ou le chlore, en cours de l'étape a). Le rapport molaire de l'oxydant sur le composé **A** ou **B** peut être entre 0,005 et 2, de préférence entre 0,01 et 1,5. L'oxydant peut être de l'oxygène pur, de l'air ou un mélange d'oxygène et d'azote.

L'étape a) peut éventuellement comprendre une étape de régénération du catalyseur en alternance avec ladite réaction catalytique. L'étape de régénération est généralement mise en œuvre en présence d'un flux comprenant de l'oxygène à une température comprise entre 100°C et 500°C.

A la fin de la mise en œuvre de l'étape a), le catalyseur est soumis à l'étape b) selon le procédé de la présente invention.

### Etape b)

Selon le présent procédé, l'étape b) comprend l'écoulement d'un gaz inerte à travers le lit catalytique. De préférence, l'étape b) est mise en œuvre à une température du lit catalytique T2 inférieure à T1. Ainsi, il n'est pas nécessaire de chauffer le lit catalytique pour éliminer l'halogénure d'hydrogène présent dans le réacteur ou le catalyseur. Pour maximiser l'élimination de l'halogénure d'hydrogène, la température du lit catalytique T2 est supérieure à 30°C. Ainsi, au début de la mise en œuvre de l'étape b), la température T2 est supérieure à 30°C. Après la mise en œuvre de l'étape a), le flux des réactifs est arrêté, la température du lit catalytique diminue de la température T1 à la température T2, inférieure à T1, et le gaz inerte est introduit dans le réacteur.

De préférence, la température du lit catalytique T2 est supérieure à 40°C, avantageusement supérieure à 50°C, de préférence supérieure à 60°C, plus préférentiellement supérieure à 70°C, en particulier supérieure à 80°C, plus particulièrement supérieure à 90°C, de manière privilégiée supérieure à 100°C.

De préférence, la température du lit catalytique T2 est inférieure à 380°C, avantageusement inférieure à 360°C, de préférence inférieure à 340°C, plus préférentiellement inférieure à 320°C, en particulier inférieure à 300°C, de manière privilégiée inférieure à 250°C.

Selon un mode de réalisation préféré, le gaz inerte est introduit dans le réacteur à une température comprise entre la température ambiante et T2, avantageusement entre la température ambiante et 50°C, en particulier le gaz inerte est introduit dans le réacteur à température ambiante.

Le passage du gaz inerte à travers le lit catalytique entraine une diminution de la température du lit catalytique T2 au cours de la mise en œuvre de l'étape b). De préférence, la température T2 diminue à une vitesse inférieure à 5°C/min au cours de la mise en œuvre de l'étape b), en particulier inférieure à 1°C/min, au cours de la mise en œuvre de l'étape b).

Selon un mode de réalisation préféré, le gaz inerte s'écoule à un débit supérieur à 0,1 ml/min par ml de catalyseur, avantageusement supérieur à 0,2 ml/min par ml de catalyseur, de préférence supérieur à 0,3 ml/min par ml de catalyseur, plus préférentiellement supérieur à 0,4 ml/min par ml de catalyseur, en particulier supérieur à 0,5 ml/min par ml de catalyseur, de manière privilégiée supérieur à 0,6 ml/min par ml de catalyseur, de manière avantageusement privilégiée supérieur à 0,7 ml/min par ml de catalyseur, de manière préférentiellement privilégiée supérieur à 0,8 ml/min par ml de catalyseur, de manière particulièrement privilégiée supérieur à 0,9 ml/min par ml de catalyseur.

Selon un mode de réalisation préféré, l'étape b) comprend une étape b1) de refroidissement du lit catalytique de la température T1 à T2 puis une étape b2) d'écoulement dudit gaz inerte à travers le lit catalytique.

De préférence, le gaz inerte est de l'azote ou de l'argon. En particulier de l'azote.

De préférence, en sortie du réacteur, le gaz inerte contient une teneur massique en CO et CO2 inférieure à 100 ppm.

### Exemples

L'appareillage utilisé comprend un réacteur tubulaire en INCONEL^{®} 600 (diamètre interne de 28 mm - longueur = 600 mm), placé verticalement dans un four électrique tubulaire. Le réacteur est équipé d'indicateurs de pression et de température (thermocouple mobile dans une gaine en inconel placée coaxialement au centre du tube). Le lit catalytique fixe est constitué d'une couche inférieure de corindon puis d'une couche de catalyseur de 180 ml et d'une couche supérieure de corindon. Le catalyseur utilisé est un catalyseur Ni-Cr/AlF₃. Avant utilisation, il est séché puis activé en présence d'un mélange d'acide fluorhydrique et d'azote, à une température comprise entre T = 320°C et T = 350°C. Les caractéristiques du catalyseur après activation sont les suivantes :
- surface BET : 38.78 m²/g ;
- composition chimique : Al :19.0 %, F : 61.2 %, Cr : 4.5 %, Ni : 4.4 %

Les réactifs sont introduits en continu à l'extrémité supérieure du réacteur et préchauffés à la température du four à travers la couche supérieure de corindon, les produits gazeux de la réaction sortent à l'extrémité inférieure du réacteur à travers une vanne de régulation de pression, le flux gazeux sortant de la vanne est analysé par chromatographie phase gaz.

### Essai 1 (invention)

La réaction est effectuée à pression atmosphérique et à une température de T = 350°C en alimentant en continu de l'HF anhydre (137.6 g.h⁻¹) et du perchloroéthylène (28.3 g.h⁻¹). La VSHG (vitesse spatiale horaire des gaz) est de 2000 h⁻¹. Le ratio molaire HF : organique est de 40.3. Après 19 h de réaction, la composition du flux organique sortant du réacteur est reprise dans le tableau 1 ci-dessous.

**[Tableau 1]**

| Composition | PER | F125 | F124 | F123 | F121+F122 | Autres |
|---|---|---|---|---|---|---|
| mol % | 29.39 | 21.68 | 25.33 | 13.55 | 4.77 | 5.28 |

Après 98 h de réaction, l'introduction des réactifs et le chauffage du four électrique sont arrêtés. De l'azote est introduit dans le réacteur à un débit de 10 l.h⁻¹ (0.9 ml.min⁻¹ par ml de catalyseur). Après 17h de balayage, la température dans le réacteur est de T = 140°C (soit une diminution à une vitesse moyenne de 12°C par heure).

### Essai 2 (comparatif)

Un essai identique à l'essai 1 (même lot de catalyseur activé) est réalisé jusqu'à l'arrêt des réactifs. Après 98 h de réaction, l'introduction des réactifs est arrêtée et le chauffage du four électrique est augmenté jusqu'à atteindre une température de 360°C. De l'azote est introduit dans le réacteur à un débit de 10 l.h⁻¹ (0.9 ml.min⁻¹ par ml de catalyseur). La température du four est maintenue à une température de T = 360°C pendant 8 heures.

### Exemple 1

Le catalyseur de l'essai 1 et le catalyseur de l'essai 2 sont régénérés de façon identique à pression atmosphérique, par un traitement à l'air (1.5 l.h⁻¹) à une température de T = 350°C pendant 72 heures. Après régénération, les catalyseurs sont analysés (tableau 2).

**[Tableau 2]**

| Surface BET (m²/g) | |
|---|---|
| Catalyseur de l'essai 1 (après régénération) | Catalyseur de l'essai 2(après régénération) |
| 25.79 | 20.03 |

Les catalyseurs de l'essai 1 et de l'essai 2 ainsi régénérés sont testés dans la réaction de fluoration du perchloroéthylène. La réaction est effectuée à pression atmosphérique et à une température de T = 350°C en alimentant en continu de l'HF anhydre (137.6 g.h⁻¹) et du perchloroéthylène (28.3 g.h⁻¹). La VSHG (vitesse spatiale horaire des gaz) est de 2000 h⁻¹. Le ratio molaire HF : organique est de 40.3. L'analyse de la composition du flux organique sortant du réacteur est également effectuée après 19h et 43h de réaction. Les résultats comparatifs sont présentés dans le tableau 3 ci-dessous.

**[Tableau 3]**

| Composition (mol%) | | PER | F125 | F124 | F123 | F121+F122 | Autres |
|---|---|---|---|---|---|---|---|
| Catalyseur de l'essai 1 | 19h | 37.97 | 10.08 | 23.56 | 16.05 | 5.59 | 6.75 |
| | 43h | 40.45 | 13.03 | 21.99 | 12.96 | 5.94 | 5.63 |
| Catalyseur de l'essai 2 | 19h | 45.89 | 8.04 | 17.19 | 14.95 | 6.48 | 7.45 |
| | 43h | 48.2 | 8.62 | 15.19 | 13.99 | 6.5 | 7.5 |

Ces résultats indiquent clairement que le traitement effectué sur le catalyseur de l'essai 2 est néfaste pour celui-ci (surface BET et activité catalytique plus faibles). Au contraire, le traitement à l'azote effectué sur le catalyseur de l'essai permet d'obtenir une meilleure activité catalytique de celui-ci après régénération. L'étape b) selon la présente invention permet d'éviter la dégradation précoce du catalyseur et d'obtenir ainsi un catalyseur plus efficace ultérieurement lorsque ce dernier est réutilisé, par exemple après régénération.

## Revendications

1. Procédé de traitement, dans un réacteur contenant un lit catalytique, d'un catalyseur solide, ledit procédé comprenant les étapes :
a) Mise en œuvre dans ledit réacteur d'une réaction catalytique en phase gazeuse à une température du lit catalytique T1 en présence d'un halogénure d'hydrogène ou générant la formation d'un halogénure d'hydrogène,
b) Ecoulement d'un gaz inerte à travers le lit catalytique à une température du lit catalytique T2 inférieure à T1; la température T2 étant supérieure à 30°C ; la température T2 diminue au cours de la mise en œuvre de l'étape b).

2. Procédé selon la revendication précédente **caractérisé en ce que** le gaz inerte introduit dans le réacteur est à une température comprise entre la température ambiante et la température T2.

3. Procédé selon l'une quelconque des revendications précédentes **caractérisé en ce que** le gaz inerte introduit dans le réacteur est à température ambiante.

4. Procédé selon l'une quelconque des revendications précédentes **caractérisé en ce que** l'halogénure d'hydrogène est le fluorure d'hydrogène ou le chlorure d'hydrogène.

5. Procédé selon l'une quelconque des revendications précédentes **caractérisé en ce que** l'halogénure d'hydrogène est sous forme anhydre.

6. Procédé selon l'une quelconque des revendications précédentes **caractérisé en ce que** la température T2 diminue à une vitesse inférieure à 1°C/min au cours de la mise en œuvre de l'étape b).

7. Procédé selon l'une quelconque des revendications précédentes **caractérisé en ce que** le gaz inerte s'écoule à un débit supérieur à 0,1 ml/min par ml de catalyseur.

8. Procédé selon l'une quelconque des revendications précédentes **caractérisé en ce que** le catalyseur est à base de carbone ou à base d'un métal sélectionné parmi le groupe consistant en Cr, Fe, Sb, Ni, Co, Zn, Al et Mn.

9. Procédé selon l'une quelconque des revendications précédentes **caractérisé en ce que** l'étape a) met en œuvre une réaction en phase gazeuse entre HF et un composé hydrohalocarboné en C₁-C₄ **A** ou l'étape a) met en œuvre une réaction de déshydrohalogénation en phase gazeuse d'un composé hydrocarboné saturé en C₁-C₄ **B** comprenant au moins un atome d'halogène pour former un composé hydrocarboné insaturé en C₁-C₄ et un halogénure d'hydrogène.

10. Procédé selon la revendication précédente **caractérisé en ce que** le composé **A** est sélectionné parmi le groupe consistant en 1,1,2-trichloroéthane, 2-chloro-1,1,1-trifluoroéthane , 1-chloro-1,1,2-trifluoroéthane, 1-chloro-1,2,2-trifluoroéthane, 1,1,1,3,3,3-hexachlorodifluoropropane, 1,1,1,3,3,3-hexachloropropane, 1,1,1,3,3-pentachloropropane, 2,2,3-trichloro-1,1,1,3,3-pentafluoropropane, 1,1,1,3,3,3-hexachlorodifluoropropane, 1,1-dichloro-2,2,3,3,3-pentafluoropropane, 1,3-dichloro-1,2,2,3,3-pentafluoropropane, 1,1,1,2,3-pentachloropropane, 1,1,2,2,3-pentachloropropane, 1,1,1,3,3-pentachloropropane, 1,2-dichloroéthylène, 1,1,2-trichloroethylène, 1,1,2,2-tétrachloroéthylène, 1,1,2-trichloro-3,3,3-trifluoropropène, l'hexafluoropropène, 1,1,3,3,3-pentafluoropropène, 1,3,3,3-tétrafluoropropène, 2-chloro-3,3,3-trifluoropropène, 1,1,2,3-tétrachloropropène, 2,3,3,3-tétrachloropropène, 1-chloro-3,3,3-trifluoropropène, 1,1,3,3-tétrachloropropène, 1,3,3,3-tétrachloropropène, 2,3-dichloro-1,1,1-trifluoropropène, 2-chloro-1,1,1,2-tétrafluoropropane ; ou
le composé **B** est sélectionné parmi le groupe consistant en 1,1-difluoroéthane, 1,1,1-trifluoroéthane, 2-chloro-1,1,1-trifluoroéthane, 1,1,1,2-tétrafluoroéthane, 1,1,2,2-tétrafluoroéthane, 1,1,1,2,2-pentafluoroéthane, 1,1,1,2-tétrafluoropropane, 1,1,1,2,3,3-hexafluoropropane, 1,1,1,3,3,3-hexafluoropropane, 1,1,1,2,3,3-hexafluoropropane, 1,1,1,2,2-pentafluoropropane, 1,1,1,2,3-pentafluoropropane, 1,1,1,3,3-pentafluoropropane, 2,3-dichloro-1,1,1-trifluoropropane, 2-chloro-1,1,1,2-tétrafluoropropane.

11. Procédé selon l'une quelconque des revendications précédentes **caractérisé en ce qu'**il comprend une étape c) de déchargement du catalyseur dudit réacteur.

12. Procédé selon l'une quelconque des revendications précédentes **caractérisé en ce que** l'étape b) comprend une étape b1) de refroidissement du lit catalytique de la température T1 à T2 puis une étape b2) d'écoulement dudit gaz inerte à travers le lit catalytique.

## Patentansprüche

1. Verfahren zur Behandlung eines festen Katalysators in einem Reaktor, der eine Katalysatorschüttung enthält, wobei das Verfahren folgende Schritte umfasst:
a) Durchführen in dem Reaktor einer katalytischen Gasphasenreaktion bei einer Katalysatorschüttungstemperatur T1 in Gegenwart eines Halogenwasserstoffs oder einer zur Bildung eines Halogenwasserstoffs führenden katalytischen Gasphasenreaktion;
b) Durchleiten von Inertgas durch die Katalysatorschüttung bei einer Katalysatorschüttungstemperatur T2 unterhalb von T1; wobei die Temperatur T2 höher als 30 °C ist; wobei die Temperatur T2 im Lauf der Durchführung von Schritt b) abnimmt.

2. Verfahren nach dem vorhergehenden Anspruch, **dadurch gekennzeichnet, dass** das in den Reaktor eingetragene Inertgas bei einer Temperatur zwischen Umgebungstemperatur und der Temperatur T2 vorliegt.

3. Verfahren nach einem der vorhergehenden Ansprüche, **dadurch gekennzeichnet, dass** das in den Reaktor eingetragene Inertgas bei Raumtemperatur vorliegt.

4. Verfahren nach einem der vorhergehenden Ansprüche, **dadurch gekennzeichnet, dass** es sich bei dem Halogenwasserstoff um Fluorwasserstoff oder Chlorwasserstoff handelt.

5. Verfahren nach einem der vorhergehenden Ansprüche, **dadurch gekennzeichnet, dass** der Halogenwasserstoff in wasserfreier Form vorliegt.

6. Verfahren nach einem der vorhergehenden Ansprüche, **dadurch gekennzeichnet, dass** die Temperatur T2 im Lauf der Durchführung von Schritt b) mit einer Geschwindigkeit von weniger als 1 °C/min abnimmt.

7. Verfahren nach einem der vorhergehenden Ansprüche, **dadurch gekennzeichnet, dass** das Inertgas mit einer Durchflussrate von mehr als 0,1 ml/min pro ml Katalysator strömt.

8. Verfahren nach einem der vorhergehenden Ansprüche, **dadurch gekennzeichnet, dass** der Katalysator auf Kohlenstoff basiert oder auf einem Metall aus der Gruppe bestehend aus Cr, Fe, Sb, Ni, Co, Zn, Al und Mn basiert.

9. Verfahren nach einem der vorhergehenden Ansprüche, **dadurch gekennzeichnet, dass** in Schritt a) eine Gasphasenreaktion zwischen HF und einer C₁-C₄-Halogenkohlenwasserstoffverbindung A durchgeführt wird oder in Schritt a) eine Gasphasen-Dehydrohalogenierungsreaktion einer gesättigten C₁-C₄-Kohlenwasserstoffverbindung B, die mindestens ein Halogenatom umfasst, zur Bildung einer ungesättigten C₁-C₄-Kohlenwasserstoffverbindung und eines Halogenwasserstoffs durchgeführt wird.

10. Verfahren nach dem vorhergehenden Anspruch, **dadurch gekennzeichnet, dass** die Verbindung A aus der Gruppe bestehend aus 1,1,2-Trichlorethan, 2-Chlor-1,1,1-trifluorethan, 1-Chlor-1,1,2-trifluorethan, 1-Chlor-1,2,2-trifluorethan, 1,1,1,3,3,3-Hexachlordifluorpropan, 1,1,1,3,3,3-Hexachlorpropan, 1,1,1,3,3-Pentachlorpropan, 2,2,3-Trichlor-1,1,1,3,3-pentafluorpropan, 1,1,1,3,3,3-Hexachlordifluorpropan, 1,1-Dichlor-2,2,3,3,3-pentafluorpropan, 1,3-Dichlor-1,2,2,3,3-pentafluorpropan, 1,1,1,2,3-Pentachlorpropan, 1,1,2,2,3-Pentachlorpropan, 1,1,1,3,3-Pentachlorpropan, 1,2-Dichlorethylen, 1,1,2-Trichlorethylen, 1,1,2,2-Tetrachlorethylen, 1,1,2-Trichlor-3,3,3-trifluorpropen, Hexafluorpropen, 1,1,3,3,3-Pentafluorpropen, 1,3,3,3-Tetrafluorpropen, 2-Chlor-3,3,3-trifluorpropen, 1,1,2,3-Tetrachlorpropen, 2,3,3,3-Tetrachlorpropen, 1-Chlor-3,3,3-trifluorpropen, 1,1,3,3-Tetrachlorpropen, 1,3,3,3-Tetrachlorpropen, 2,3-Dichlor-1,1,1-trifluorpropen, 2-Chlor-1,1,1,2-tetrafluorpropan ausgewählt wird; oder
Verbindung B aus der Gruppe bestehend aus 1,1-Difluorethan, 1,1,1-Trifluorethan, 2-Chlor-1,1,1-trifluorethan, 1,1,1,2-Tetrafluorethan, 1,1,2,2-Tetrafluorethan, 1,1,1,2,2-Pentafluorethan, 1,1,1,2-Tetrafluorpropan, 1,1,1,2,3,3-Hexafluorpropan, 1,1,1,3,3,3-Hexafluorpropan, 1,1,1,2,2,3-Hexafluorpropan, 1,1,1,2,2-Pentafluorpropan, 1,1,1,2,3-Pentafluorpropan, 1,1,1,3,3-Pentafluorpropan, 2,3-Dichlor-1,1,1-trifluorpropan, 2-Chlor-1,1,1,2-tetrafluorpropan ausgewählt wird.

11. Verfahren nach einem der vorhergehenden Ansprüche, **dadurch gekennzeichnet, dass** es einen Schritt c) zum Austragen des Katalysators aus dem Reaktor umfasst.

12. Verfahren nach einem der vorhergehenden Ansprüche, **dadurch gekennzeichnet, dass** Schritt b) einen Schritt b1) des Abkühlens der Katalysatorschüttung von der Temperatur T1 auf T2 und dann einen Schritt b2) des Durchleitens des Inertgases durch die Katalysatorschüttung umfasst.

## Claims

1. Process for treating, in a reactor containing a catalytic bed, a solid catalyst, said process comprising the steps of:
a) implementing, in said reactor, a gas-phase catalytic reaction at a catalytic bed temperature T1 in the presence of a hydrogen halide or giving rise to the formation of a hydrogen halide,
b) causing an inert gas to flow through the catalytic bed at a catalytic bed temperature T2 that is lower than T1, the temperature T2 being greater than 30°C, the temperature T2 decreasing during the implementation of step b).

2. Process according to the preceding claim, **characterized in that** the inert gas introduced into the reactor is at a temperature of between ambient temperature and the temperature T2.

3. Process according to either one of the preceding claims, **characterized in that** the inert gas introduced into the reactor is at ambient temperature.

4. Process according to any one of the preceding claims, **characterized in that** the hydrogen halide is hydrogen fluoride or hydrogen chloride.

5. Process according to any one of the preceding claims, **characterized in that** the hydrogen halide is in anhydrous form.

6. Process according to any one of the preceding claims, **characterized in that** the temperature T2 decreases at a rate of less than 1°C/min during the implementation of step b).

7. Process according to any one of the preceding claims, **characterized in that** the inert gas flows at a flow rate of greater than 0.1 ml/min per ml of catalyst.

8. Process according to any one of the preceding claims, **characterized in that** the catalyst is based on carbon or based on a metal selected from the group consisting of Cr, Fe, Sb, Ni, Co, Zn, Al and Mn.

9. Process according to any one of the preceding claims, **characterized in that** step a) implements a gas-phase reaction between HF and a C₁-C₄ halohydrocarbon compound A or step a) implements a gas-phase dehydrohalogenation reaction of a saturated C₁-C₄ hydrocarbon compound B comprising at least one halogen atom to form an unsaturated C₁-C₄ hydrocarbon compound and a hydrogen halide.

10. Process according to the preceding claim, **characterized in that** the compound A is selected from the group consisting of 1,1,2-trichloroethane, 2-chloro-1,1,1-trifluoroethane, 1-chloro-1,1,2-trifluoroethane, 1-chloro-1,2,2-trifluoroethane, 1,1,1,3,3,3-hexachlorodifluoropropane, 1,1,1,3,3,3-hexachloropropane, 1,1,1,3,3-pentachloropropane, 2,2,3-trichloro-1,1,1,3,3-pentafluoropropane, 1,1,1,3,3,3-hexachlorodifluoropropane, 1,1-dichloro-2,2,3,3,3-pentafluoropropane, 1,3-dichloro-1,2,2,3,3-pentafluoropropane, 1,1,1,2,3-pentachloropropane, 1,1,2,2,3-pentachloropropane, 1,1,1,3,3-pentachloropropane, 1,2-dichloroethylene, 1,1,2-trichloroethylene, 1,1,2,2-tetrachloroethylene, 1,1,2-trichloro-3,3,3-trifluoropropene, hexafluoropropene, 1,1,3,3,3-pentafluoropropene, 1,3,3,3-tetrafluoropropene, 2-chloro-3,3,3-trifluoropropene, 1,1,2,3-tetrachloropropene, 2,3,3,3-tetrachloropropene, 1-chloro-3,3,3-trifluoropropene, 1,1,3,3-tetrachloropropene, 1,3,3,3-tetrachloropropene, 2,3-dichloro-1,1,1-trifluoropropene, 2-chloro-1,1,1,2-tetrafluoropropane; or
the compound B is selected from the group consisting of 1,1-difluoroethane, 1,1,1-trifluoroethane, 2-chloro-1,1,1-trifluoroethane, 1,1,1,2-tetrafluoroethane, 1,1,2,2-tetrafluoroethane, 1,1,1,2,2-pentafluoroethane, 1,1,1,2-tetrafluoropropane, 1,1,1,2,3,3-hexafluoropropane, 1,1,1,3,3,3-hexafluoropropane, 1,1,1,2,2,3-hexafluoropropane, 1,1,1,2,2-pentafluoropropane, 1,1,1,2,3-pentafluoropropane, 1,1,1,3,3-pentafluoropropane, 2,3-dichloro-1,1,1-trifluoropropane, 2-chloro-1,1,1,2-tetrafluoropropane.

11. Process according to any one of the preceding claims, **characterized in that** it comprises a step c) of unloading the catalyst from said reactor.

12. Process according to any one of the preceding claims, **characterized in that** step b) comprises a step b1) of cooling the catalytic bed from the temperature T1 to T2, and then a step b2) of causing said inert gas to flow through the catalytic bed.
